# EUROPÄISCHE PATENTANMELDUNG

(11) **EP 3 932 457 A1**
(43) Veröffentlichungstag der Anmeldung: **05.01.2022**
(21) Anmeldenummer: 20183767.1
(22) Anmeldetag: 02.07.2020
(51) Int. Cl.: A61M 16/04, A61H 23/00, A61M 16/00, A61M 16/10, A61M 25/00, A61M 1/00

(54) **TRACHEALKATHETER, INTUBATIONSSCHLAUCH UND BEHANDLUNGSVORRICHTUNG**

(71) Anmelder: Carag AG, 6340 Baar (CH)
(72) Erfinder: Larsson, Michael, 6302 Zug (CH); Christen, Lukas, 6004 Luzern (CH); Bernhard, Jérôme, 8003 Zürich (CH)
(74) Vertreter: Grünecker Patent- und Rechtsanwälte PartG mbB

(57) **Zusammenfassung**

Die vorliegende Erfindung betrifft einen Trachealkatheter (60) mit einem in die Lunge einbringbaren distalen Ende (62) und einem dabei extrakorporal angeordneten proximalen Ende (66), wobei zur verbesserten Lösung von Schleim in der Lunge im Bereich des distalen Endes (62) ein das distale Ende (62) umgebendes Medium physikalisch anregende Erregereinrichtung (19) vorgesehen ist. Die vorliegende Erfindung betrifft ferner einen Intubationsschlauch (2) mit einem ersten Lumen (8) für einen Gasaustausch und einem zweiten Lumen (14) für die Steuerungen eines Cuffs (12), der von einem distalen Ende (10) des ersten Lumens (8) überragt ist, wobei zur verbesserten Lösung von Schleim eine dem distalen Ende zugeordnete Erregereinrichtung (19) vorgesehen ist, die ein die Erregereinrichtung (19) umgebendes Medium physikalisch anregt. Des Weiteren betrifft die vorliegende Erfindung eine Vorrichtung zur Behandlung von Lungenkrankheiten mit einem Beatmungsgerät, das mit einem ersten Lumen (8) eines Intubationsschlauches (2) verbindbar ist, einer Saugpumpe (32), die an zumindest einer Ventileinrichtung (24) angeschlossen ist, die die Saugpumpe (32) mit einer Schnittstelle (20) für einen Saugkanal eines Katheters verbindet, der im Bereich seines distalen Endes mit einer Erregereinrichtung versehen ist, und einer Steuerung (46), die steuerungsmäßig mit der mindestens einen Ventileinrichtung (24), der Saugpumpe (32) und der Erregereinrichtung (19) verbunden ist.

## Beschreibung

Die vorliegende Erfindung betrifft die Behandlung von Lungenkrankheiten und schlägt hierzu einen Trachealkatheter, einen verbesserten Intubationsschlauch und eine dazu angepasst ausgebildete Behandlungsvorrichtung für die Behandlung von Lungenkrankheiten vor.

Lungenerkrankungen, beispielsweise infektiöser Art, bedürfen mitunter der Beatmung des Patienten. Hierzu wird der Patient intubiert und über einen konventionellen Intubationsschlauch beatmet. Lungenerkrankungen können aber auch zu einer Überproduktion von Sekreten in der Lunge führen, die die zentrale Funktion der Lunge behindern, so dass Gasaustausch trotz künstlicher Beatmung nur unzureichend möglich ist. Beispiele für Erkrankungen solcher Art können Lungenentzündungen, Lungenödeme, COPD, cystische Fibrose oder andere schleimbildende oder flüssigkeitsbildende Lungenkrankheiten sein.

Die Verschleimung der Lunge stellt dabei ein erhebliches Problem dar. Schleim vermindert den Gasaustausch und beeinträchtigt dementsprechend die Funktion der Lunge. Dabei besteht auch das Problem, dass sich Schleim in den Alveolen der Lunge befinden kann. Man ist bemüht, in Alveolen befindlichen Schleim zu den Bronchien zu fördern, da dieser dort leichter entfernt werden kann.

Dazu gibt es bereits Lösungsvorschläge, die darauf abzielen, die bei der Beatmung eingebrachte Luft stoßweise einzubringen, bevorzugt in kurzen Frequenzen, um so ein Ablösen des Schleims zu begünstigen. Unter dem Stichwort high-frequency chest wall oscillation ist eine Behandlungsmethode bekannt, bei welcher eine aufblasbare Weste um den Patienten gelegt wird, in der Luftdruck vibriert. Dadurch soll versucht werden, das Ablösen von Schleim in der Lunge und damit den Gasaustausch zu verbessern.

Ungeachtet der obigen Problematik kann ein Medium innerhalb der Lunge, d.h. Schleim oder koaguliertes Blut nicht immer zuverlässig abgesaugt werden. Im Durchmesser kleinere Drainageschläuche können größere Ansammlungen von Schleim oder koaguliertem Blut nicht fördern. So müssen größere Schläuche eingesetzt werden, was beispielweise im Falle einer Beatmung auf Kosten des freien Durchmessers eines ersten Lumens eines Intubationsschlauches geht, durch welches der Patient beim Intubieren mit dem Schlauch beatmet wird.

Hier will die vorliegende Erfindung Abhilfe schaffen und einen Trachealkatheter zur Entfernung von Schleim und anderen korpuskulären Bestandteilen aus der Lunge vorschlagen. Die vorliegende Erfindung will ferner einen Intubationsschlauch vorschlagen, der in verbesserter Weise für die Behandlung von Lungenkrankheiten angepasst ist. Ferner will die vorliegende Erfindung eine verbesserte Vorrichtung zur Behandlung von Lungenerkrankungen vorschlagen.

Gemäß Ihrem ersten Aspekt schlägt die vorliegende Erfindung einen Trachealkatheter mit den Merkmalen von Anspruch 1 vor. Dieser Trachealkatheter hat ein in den Körper einbringbares distales Ende. Das distale Ende reicht üblicherweise bis zu den Bronchien, idealerweise auch bis in einen Lungenflügel hinein. Der Trachealkatheter hat ein bei dieser Verlegung des Katheters extrakorporal angeordnetes proximales Ende. Im Bereich des distalen Endes ist eine Erregereinrichtung vorgesehen. Diese Erregereinrichtung ist dazu ausgebildet, ein im Bereich des distalen Endes vorgesehenes Medium physikalisch anzuregen.

Im Rahmen der Beschreibung der vorliegenden Erfindung bezeichnet "distal" den bei der Benutzung des Trachealkatheters respektive Intubationsschlauchs im Körper liegenden Endbereich des Trachealkatheters respektive Intubationsschlauches, wohingegen "proximal" das aus dem Körper herausstehende Ende des Trachealkatheters respektive Intubationsschlauches bezeichnet.

Als Trachealkatheter im Sinne der vorliegenden Erfindung ist auch ein Bronchial-Katheter oder ein Bronchoalveolar-Katheter zu verstehen

Die Erregereinrichtung nach der vorliegenden Erfindung kann beispielsweise ein elektrisch angetriebener Vibrator, eine Schallquelle oder dergleichen sein. Eine Erregereinrichtung ist insbesondere eine elektrische Einrichtung, die durch Bestromung in Schwingung versetzt werden kann. Die Erregereinrichtung kann für sich vibrieren und diese Vibration durch unmittelbaren körperlichen Kontakt mit dem Fluid oder dem Körpergewebe in das Fluid oder in das Körpergewebe einleiten. Ergänzend oder alternativ kann die Erregereinrichtung auch Schallwellen absenden, die auf das umgebene Fluid oder Körpergewebe auftreffen und dieses anregen.

Als Erregereinrichtung im Sinne der vorliegenden Erfindung ist jede Einrichtung zu verstehen, die an das im Bereich des distalen Endes vorgesehene und in der Lunge befindliche Fluid so anregen, dass sich dieses leichter von der inneren Oberfläche der Lunge löst und dementsprechend aus der Lunge leichter ausgebracht werden kann.

So schlägt die vorliegende Erfindung einen Trachealkatheter vor, mit dem die Erregereinrichtung in die Lunge, bevorzugt tief in die Lunge bis in die Bronchien oder die Lungenflügel eingebracht werden kann, um dort Fluid anzuregen, sodass sich dieses leichter löst und ausgebracht werden kann.

Gemäß einer bevorzugten Weiterbildung der vorliegenden Erfindung ist an dem proximalen Ende des Trachealkatheters ein Schlauchanschluss vorgesehen, der mit einem in dem Trachealkatheter ausgebildeten und bis zu dem distalen Ende reichenden Katheterlumen kommuniziert. Der Trachealkatheter ist dementsprechend als Schlauchkatheter ausgebildet. So bietet der Katheter für sich die Möglichkeit, das durch Erregung gelöste Fluid unmittelbar durch Absaugung aus der Lunge zu entfernen.

Im Hinblick auf eine effektive und möglichst tief in die Lunge reichende Absaugung sollte ein solcher Trachealkatheter mit einem Katheterlumen einen Außendurchmesser von zwischen 2 mm und 10 mm, bevorzugt von zwischen 2 und 5 mm haben.

Im Hinblick auf eine möglichst genaue Positionierung des Trachealkatheters und insbesondere der Erregereinheit an einer bestimmten Stelle innerhalb der Lunge z.B. in einen Lungenflügel ist der Trachealkatheter vor dem Einführen in die Lunge mit Führungsdrähten versehen. Dabei ist dem Trachealkatheter ein bis zu dem freien Ende des Trachealkatheters reichender Führungsdraht zugeordnet, der aus dem Trachealkatheter herausziehbar sein kann. Ein solcher Führungsdraht wird beispielsweise bei minimalinvasiven kardiovaskulären Eingriffen genutzt, um einen vaskulär eingeführten Schlauch in die gewünschte Position zu bringen bzw. innerhalb des Körpers auszurichten. An diesen Vorbildern orientiert sich die hier diskutierte Weiterbildung.

Der Führungsdraht kann abhängig von seinem Durchmesser in die Wand des Trachealkatheters eingebracht oder innerhalb des Trachealkatheters verlegt sein und mit dem Trachealkatheter verbunden sein. Der Trachealkatheter hat einen relativ geringen Außendurchmesser, um möglichst tief in den linken bzw. rechten Lungenflügel eingebracht zu werden. Der Trachealkatheter sollte ausreichend steif sein, um ein Kollabieren des Katheterlumens aufgrund des Unterdrucks zu verhindern. Der effektive Strömungsdurchmesser der Katheterlumens sollte im Hinblick auf einen möglichst geringen Strömungswiderstand möglichst groß sein. Die Auswahl des Führungsdrahtes und die Anordnung in dem Trachealkatheter sollte diesen Vorgaben bestmöglich entsprechen. Der Trachealkatheter kann zur Erhöhung der Knicksteifigkeit gebraided, d.h. mit einer umfänglichen dünnwandigen Versteifung versehen sein. Die Eigensteifigkeit des Trachealkatheters sollte beispielsweise durch die Materialbeschaffenheit des den Trachealkatheter bildenden Materials und/oder beispielsweise in eine Kunststoffmatrix eingebrachte Versteifungselemente so ausgebildet sein, dass der Trachealkatheter insbesondere beim Saugen durch das Katheterlumen nicht kollabiert und einen hinreichenden Durchlass von Fluid während des Absaugens ermöglicht.

Ein optischer Leiter im Trachealkatheter ist ferner denkbar, um die Lage des Trachealkatheters und der Führungsdrähte zu kontrollieren. Dieser optische Leiter sollte bis zu dem distalen Ende des Trachealkatheters reichen, um auch eine Inspektion der Lunge, insbesondere der rechten und linken Hauptbronchien zu ermöglichen. Dazu sollte der Katheter an seinem distalen Ende mit einer Lichtquelle versehen sein.

Alternativ oder zusätzlich zu den Führungsdrähten kann der Trachealkatheter einen gebogenen elastischen Abschnitt, insbesondere am freien Ende des Trachealkatheters, aufweisen. Der elastische Abschnitt ist vorteilhaft, da er beim Einführen durch einen Kanal oder Lumen verformbar ist, aber seine gebogene Form wieder einnimmt, wenn der Trachealkatheter den Bereich des Kanals oder Lumens verlassen hat. Üblicherweise ist lediglich das distale Ende des Trachealkatheters gebogen. Eine Biegung bedeutet hierbei, dass der gebogene elastische Abschnitt relativ zu dem üblichen geradlinigen Verlauf des Trachealkatheters abweicht, sodass der Trachealkatheter eine einseitig abgehende Krümmung an seinem Ende hat. Eine gebogene Form an dem distalen Ende des Trachealkatheters kann für die Orientierung und/oder Positionierung verwendet werden.

Im Hinblick auf eine möglichst schonende Einbringung des Trachealkatheters z.B. in einen Lungenflügel wird gemäß einer bevorzugten Weiterbildung der vorliegenden Erfindung vorgeschlagen, den Trachealkatheter mit einer Beschichtung, insbesondere einer antihaftenden hydrophilen oder hydrophoben Beschichtung, zu versehen, die den Reibkoeffizienten an der Innen- und/oder Außenumfangsfläche des Trachealkatheter vermindert. Eine Beschichtung in diesem Sinne ist eine permanent mit dem Trachealkatheter verbundene Beschichtung und verhindert das Verkleben des Schlauches durch proteinhaltige Sekrete. Zusätzlich kann unmittelbar vor der Benutzung der Trachealkatheter durch Aufsprühen oder Einreiben mit einem reibungsvermindernden Fluid für das Einführen in den Lungenflügel vorbereitet werden. Eine Beschichtung an der Außenumfangsfläche verbessert das Eindringen in die Luftröhre bzw. Teile der Lunge. Eine Beschichtung an der Innenumfangsfläche des Katheterlumens verbessert das Abfördern von Sekret durch das Katheterlumen.

Zur externen Kontrolle des Einführens und der Lage des Trachealkatheters hat dieser bevorzugt eine detektierbare Markierung. Die Markierung ist beispielsweise nicht-invasiv im Körper angeordnet, wobei ebenfalls der Einsatz eines Bronchoskops denkbar ist, welches bereits das durch den erfindungsgemäßen Trachealkatheter ausgebildet sein kann. Bei dieser Markierung kann es sich beispielsweise um einen metallischen Ring handeln, der über ein Röntgengerät detektiert werden kann, wodurch sich Informationen zur Lage des Trachealkatheters in der Lunge ableiten lassen.

Gemäß einer weiteren bevorzugten Ausgestaltung der vorliegenden Erfindung hat der Trachealkatheter zumindest eine an dem Umfang des Trachealkatheter vorgesehene Drainageöffnung, die mit dem Katheterlumen kommuniziert. In der Regel sind über die Länge des Trachealkatheters eine Vielzahl von Drainageöffnungen vorgesehen, die jeweils auf dem Umfang des Trachealkatheter verteilt sind und mit dem mit dem Katheterlumen kommunizieren. Dadurch wird sichergestellt, dass der Trachealkatheter auch bei einer Verstopfung einer einzelnen Drainageöffnung weiterhin Flüssigkeit aus der Lunge durch das Katheterlumen absaugen bzw. durch dieses in die Lunge einbringen kann.

Gemäß einem nebengeordneten Aspekt schlägt die vorliegende Erfindung einen verbesserten Intubationsschlauch vor. Dieser erfindungsgemäße Intubationsschlauch hat ein erstes Lumen für einen Gasaustausch und ein zweites Lumen für die Steuerung eines Cuffs, der von einem distalen Ende des ersten Lumens überragt ist und zur Abdichtung des Intubationsschlauchs in der Trachea angepasst ausgebildet ist. Insoweit unterscheidet sich der erfindungsgemäße Intubationsschlauch nicht von einem üblichen Intubationsschlauch für die künstliche Beatmung.

Der erfindungsgemäße Intubationsschlauch hat darüber hinaus im Bereich seines distalen Endes eine Erregereinrichtung, die ein die Erregereinrichtung umgebendes Medium anregt. Die Erregereinrichtung kann unmittelbar mit dem Material des Intubationsschlauches verbunden sein, welches das erste und/oder das zweite Lumen ausbildet.

Damit bietet der erfindungsgemäße Intubationsschlauch die Möglichkeit, bei der Beatmung eines Patienten auch in der Lunge befindlichen Schleim durch die Erregereinrichtung abzulösen und für den Abtransport aus der Lunge vorzubereiten. Der Abtransport kann durch Abhusten oder einen separaten Absaugkatheter erfolgen. Der Abtransport kann aber auch durch einen Drainageschlauch erfolgen, der beispielsweise in ein drittes Lumen des Intubationsschlauches eingeführt ist und bis zu dem distalen Ende des Intubationsschlauches, gegebenenfalls auch darüber hinaus und damit tiefer bis in die Lunge reichen kann.

Im Hinblick auf eine möglichst konzentrierte Ablösung von Schleim bei gleichzeitiger Abförderung insbesondere durch das Katheterlumen wird gemäß einer bevorzugten Weiterbildung der vorliegenden Erfindung vorgeschlagen, dass die Erregereinrichtung an dem zuvor beschriebenen Katheter vorgesehen ist, welcher als beweglicher Katheter in dem Intubationsschlauch angeordnet ist. Damit kann der Katheter über das distale Ende des ersten Lumens hinaus in die Lunge eingebracht werden. Der Intubationsschlauch hat für die Führung des Trachealkatheters bevorzugt ein drittes Lumen, dessen Innendurchmesser zur Führung des Trachealkatheters angepasst ausgebildet ist. Der erfindungsgemäße Intubationsschlauch kann mehrere Trachealkatheter in dieser Weise in sich aufnehmen und führen, wobei bevorzugt zwei Trachealkatheter mit Katheterlumen vorgesehen sind, die jeweils bis in einen der Lungenflügel hineingeführt werden können, um dort durch Erregung jeweils Schleim zu lösen und durch Absaugen aus der Lunge abzufördern.

Das erste Lumen endet auch bei dem erfindungsgemäßen Intubationsschlauch bevorzugt und wie an sich bekannt in der Trachea, üblicherweise im unteren Ende der Trachea. Der Trachealkatheter überragt dieses erste Lumen und ist bevorzugt hinsichtlich seiner Länge und seines Durchmessers so angepasst ausgebildet, dass der Trachealkatheter in einen der Lungenflügel (bronchus principalis dexter bzw. sinister) eingebracht werden kann. Der Trachealkatheter kann auch dem Spülen der Lunge dienen, so dass durch die Erregereinheit gelöster Schleim mit Salzlösungen, beispielsweise mit Enzymen versetzt, verflüssigt und durch das Katheterlumen abgefördert wird. Denkbar ist ebenfalls der Einsatz zur Therapie mittels Medikamente. Der Trachealkatheter sollte möglichst tief bis in die Lunge hineinragen. Der bzw. die Trachealkatheter sind über die ihnen zugeordneten Katheterlumen mit einer Saugpumpe und einer optionalen Spülpumpe verbunden.

Eine Spülpumpe ist wahlweise nutzbar, da es denkbar erscheint, den Spülvorgang ohne Spülpumpe lediglich mit Hilfe der Gravitation durchzuführen. Der Trachealkatheter kann über das ihm zugeordnete Katheterlumen wahlweise benutzt werden, um den linken oder den rechten Lungenflügel zu spülen.

Dieses Lumen kann dabei alternativ auch zumindest teilweise durch einen Schlauchkörper gebildet sein, der das erste und das zweite Lumen ausbildet und von dem ein Schlauchstück distal abgeht, das fest mit dem Schlauchkörper verbunden ist. Dieser Schlauchkörper verwirklicht die für die Beatmung notwendigen Elemente.

Die Erregereinrichtung befindet sich bevorzugt auf Höhe des distalen Endes des Katheterlumens, also desjenigen Teils des Intubationsschlauches, der zum Absaugen ggf. zum Spülen vorgesehen ist.

Dabei lässt sich die Erfindung von der Vorstellung leiten, dass üblicherweise lediglich ein Lungenflügel gespült wird, wohingegen der andere Lungenflügel zu dieser Zeit nicht gespült wird, um den Gasaustausch aufrecht zu erhalten. Die dazu vorgesehenen zwei Trachealkatheter oder das bzw. die zuvor erwähnten Schlauchstücke können jeweils mit einem Cuff versehen sein, der den Zugang zu dem entsprechenden Lungenflügel abdichtet, so dass hinter dem Cuff der Lungenflügel von effektiv von Schleim befreit und ggf. gespült werden kann, ohne dass dies den anderen Lungenflügel beeinträchtigt. Ein Cuff im Sinne der Erfindung kann aufgeblasen werden und dann die Luftröhre abdichten und auch beispielsweise das Risiko einer Aspiration vermindern.

In einer Ausführungsform ist der zumindest eine Trachealkatheter in seiner Verlaufsrichtung bzw in Verlaufsrichtung des dritten Lumens axial verschiebbar und vorzugsweise zusätzlich rotierbar. Dies ist von Vorteil, damit der Trachealkatheter möglichst kontrolliert im betroffenen Bereich der Lunge platziert werden kann.

Beim Spülen wird in der Regel vor, nach oder bei dem Betrieb der Erregereinrichtung zum Lösen von Schleim zunächst Spülflüssigkeit über das Katheterlumen des Trachealkatheters eingeleitet. Danach wird die Spülflüssigkeit durch Absaugen durch das identische Katheterlumen wieder aus dem Lungenflügel entfernt. Das Spülen stellt dabei keinen Konflikt zu der Beatmung dar. Die Beatmung erfolgt mit einem Gasvolumenstrom von beispielsweise +/- 60 Liter pro Minute bei einem Druck von etwa 40 mbar. Es ist dabei denkbar, dass für das Spülen beispielsweise ein Volumenstrom von etwa 1 Liter pro Minute ausreichend ist. Das Spülen erfolgt dabei einerseits wechselweise zwischen den beiden Lungenflügeln und in der Regel intermittierend. Nach einem Spül- und Absaugzyklus wird der Lungenflügel über einen gewissen Zeitraum nicht mehr gespült.

Im Rahmen der Behandlung wird die Beatmung üblicherweise wie allgemein bekannt kontinuierlich durchgeführt. Dieser Beatmung überlagert werden Absaug- bzw. Spülzyklen, bei denen die Erregereinrichtung betrieben wird und bei denen über jeweils einen der Katheterlumen oder der Schlauchstücke Spülflüssigkeit in einen Lungenflügel eingebracht und gegebenenfalls nach einer gewissen Einwirkzeit ggf. bei darauf angepasstem Betrieb der Erregereinrichtung wieder abgesogen wird. Als Spülflüssigkeit kann beispielsweise Kochsalzlösung zum Einsatz kommen, die in der Lunge befindlichen Schleim löst, so dass dieser beim Absaugen der Spülflüssigkeit zusammen mit der Spülflüssigkeit aus der Lunge entfernt werden kann. Die Spülflüssigkeit kann zusätzlich Enzyme, Antibiotika, Virostatika, Antiseptika oder andere geeignete entzündungshemmende Medikamente enthalten.

Hierdurch wird eine verbesserte Beatmung bewirkt. Der Gasaustausch in der Lunge ist zu einem reduzierten Anteil von Sekret in der Lunge behindert, sodass die Effektivität der Beatmung gesteigert wird.

Die erfindungsgemäße Vorrichtung hat in an sich bekannter Weise ein Beatmungsgerät, das mit einem ersten Lumen des Intubationsschlauches verbindbar ist. Die Vorrichtung hat eine Saugpumpe und optional eine Spülpumpe, die an wenigstens einer Ventileinrichtung, beispielsweise an einem 3/3 Wegeventil oder zumindest zwei Einzelventilen, angeschlossen sind. Die Ventileinrichtung verbindet die Saugpumpe mit einer Schnittstelle für ein Katheterlumen des Katheters. So kann über die Saugpumpe durch das Katheterlumen bis zu dem distalen Ende des Katheters ein Unterdruck zum Absaugen von Schleim aus der Lunge erzeugt werden. Der Katheter hat im Bereich seines distalen Endes die zuvor bereits diskutierte Erregereinrichtung.

Die erfindungsgemäße Vorrichtung hat eine Steuerung, die steuerungsmäßig mit der wenigstens einen Ventileinrichtung, der Saugpumpe und ggf. der optionalen Spülpumpe verbunden ist. Die Steuerung ist auch steuerungsmäßig mit der Erregereinrichtung verbunden.

So kann die Steuerung durch Stellen der Ventileinrichtung und Ansteuern der Saugpumpe und der optionalen Spülpumpe bzw. durch Betreiben der Erregereinrichtung die Abfolge von Lösen des Schleims, Saugen und ggf. Spülen beispielsweise in der Lunge steuern. Auf eine Spülpumpe kann beispielsweise verzichtet werden, wenn die Spülflüssigkeit mittels Gravitation in die Lunge eingeleitet wird, oder ganz auf den Einsatz von Spülflüssigkeit verzichtet wird.

Die Ventileinrichtung ist bevorzugt dazu ausgebildet drei Mechanismen zuzulassen, nämlich um (i) Spülen zu können, (ii) Saugen zu können oder (iii) einen Durchfluss zu verhindern, wozu das oder die Ventile verschlossen werden können. Sie verbindet die Saug- und die optionale Spülpumpe mit einer Schnittstelle für das Katheterlumen des wenigstens einen Trachealkatheters bzw. für das dritte Lumen. Die Ventileinrichtung kann die Saug- und Spülpumpe mit der jeweiligen Schnittstelle wahlweise verbinden, sodass entweder gespült oder durch die gleiche Leitung drainiert, d.h. abgesaugt werden kann. Die entsprechende Ventileinrichtung ist ferner so ausgebildet, dass sie das Katheterlumen proximal verschließen kann, sodass dessen Strömungsweg die Beatmung durch das Beatmungsgerät nicht beeinflusst.

Die erfindungsgemäße Vorrichtung hat bevorzugt einen in einem Leitungsweg der Spülpumpe angeschlossenen Drucksensor, dessen Signal in der Steuerung verarbeitet wird. Dieser Drucksensor lässt beispielsweise Blockaden erkennen, wenn Spülflüssigkeit zwar durch die Spülpumpe gepumpt wird, allerdings nicht bis zu dem Ende des zugeordneten Trachealkatheters gelangt. Dieses Ereignis kann dazu führen, dass die Steuerung ein optisches oder akustisches Signal absetzt, welches auf eine Störung hinweist. Ein entsprechendes Ereignis kann auch dazu führen, dass die Saug- und Spülfunktion automatisiert und/oder der Betrieb der Erregereinrichtung verändert, insbesondere intensiviert wird.

Bevorzugt hat die Vorrichtung einen in einem Leitungsweg der Saugpumpe angeschlossenen Durchflussmesser, dessen Signal in der Steuerung verarbeitet wird. Der Durchflussmesser ermittelt das abgesaugte Volumen und dient der Überwachung, damit die Beatmung nicht beeinträchtigt wird. Ferner gibt dieser Aufschluss über auffällige Verstopfungen im Trachealkatheter. Dabei befindet sich der Durchflussmesser üblicherweise in Strömungsrichtung hinter einem Auffangbehälter für das abgesaugte Sekret aus der Lunge. So misst der Durchflussmesser das Volumen der aus der Lunge abgesaugten Luft ohne die flüssigen Bestandteile und kann somit Auskünfte über den Heilungsverlauf geben. Des Weiteren kann über einen solchen Durchflussmesser eine Fehlfunktion erkannt werden, wenn durch den Drainageschlauch ein zu hohes Volumen aus der Lunge abgeleitet wird, was den Verdacht begründet, dass die zur Beatmung eingebrachte Luft unmittelbar durch das Katheterlumen abgesaugt wird.

Die Signale des Durchflussmessers bzw. des Drucksensors können dabei nicht nur Eingang in die Steuerung für das Spülen und Absaugen finden. Die entsprechenden Signale können auch ausgewertet werden, um etwaige Verstopfung insbesondere im distalen Endbereich des Trachealkatheters durch verstärkte Erregung über die Erregereinrichtung aufzulösen. Mangelnder Abfluss durch das Kathetervolumen kann in einer stärkeren Ansammlung von korpuskulären Bestandteilen am Eingang des Trachealkatheters begründet sein, die durch die verstärkte Betätigung der Erregereinrichtung beschleunigt aufgelöst werden kann.

Der zuvor erwähnte Auffangbehälter ist dabei wie ein üblicher Sekret-Auffangbehälter ausgebildet, wie er beispielsweise bei der Vakuum-Therapie zum Einsatz kommt. Zu jedem, je einem Lungenflügel zugeordneten Trachealkatheter kann ein einzelner Saugbehälter vorgesehen sein, sodass sich aus den jeweils individuell angesammelten Volumina an Sekret auch eine Aussage über den Zustand der einzelnen Lungenflügel ableiten lässt.

Weiter bevorzugt weist der Intubationsschlauch einen Drucksensor auf, insbesondere distal im Bereich des Cuffs. Mit einem solchen Drucksensor kann der Druck des Cuffs oder der Druck der durchströmenden Flüssigkeit oder des Gases im Schlauch oder der Druck in der Lunge kontrolliert werden.

Ferner betrifft die vorliegende Erfindung ein Verfahren zur Behandlung von Lungenkrankheiten mit einem Trachealkatheter der vorstehend beschriebenen Art. Bei der Behandlung wird das distale Ende des Trachealkatheters in die Lunge eingebracht. Durch Betrieb der Erregereinheit wird das Medium in der Lunge angeregt. Das hierdurch gelöste oder zerkleinerte Medium wird aus der Lunge entfernt. Dabei wird üblicherweise durch das Katheterlumen oder einen separaten Drainageschlauch das gelöste Medium, insbesondere der gelöste Schleim oder zerkleinerte koagulierte Blut, aus der Lunge abgesaugt. Gegebenenfalls kann auch nach obiger Maßgabe gespült werden. Dabei kann der Betrieb der Erregereinheit kontinuierlich oder zyklisch und im Wechsel mit dem Absaugen und/oder Spülen erfolgen.

Dabei sind Spülzyklen möglich, bei denen Spülflüssigkeit in einen Lungenflügel eingebracht und gegebenenfalls nach einer gewissen Einwirkzeit und/oder Erregungszeit wieder abgesogen wird. Als Spülflüssigkeit kann beispielsweise Kochsalzlösung, eine enzymhaltige Flüssigkeit, antibiotische- oder virostatische Flüssigkeiten, entzündungshemmende Flüssigkeiten, schleimlösende Flüssigkeiten oder andere Medikamente zum Einsatz kommen, die in der Lunge befindlichen Schleim lösen, so dass dieser beim Absaugen der Spülflüssigkeit zusammen mit der Spülflüssigkeit aus der Lunge entfernt werden kann. Die Erregereinheit kann dabei auch gezielt angewandt werden, um bei der Gabe einer mit Medikamenten versetzten Flüssigkeit die Wirkung des Wirkstoffes am distalen Ende zu erhöhen. Die Erregung durch die Erregereinheit kann genutzt werden, um den Wirkstoff besser zu verteilen und/oder mehrere Komponenten eines Wirkstoffes zu vermischen oder besser in der Tiefe der Lunge zu applizieren. Zusätzlich kann die Spülflüssigkeit genutzt werden um Vibrationen und/oder Schallwellen tiefer in die Lunge zu transportieren/übertragen.

Weitere Einzelheiten und Vorteile der vorliegenden Erfindung ergeben sich aus der nachfolgenden Beschreibung von Ausführungsbeispielen in Verbindung mit der Zeichnung. In dieser zeigen:
- Figur 1: eine schematische Ansicht eines Intubationsschlauches;
- Figur 2: eine beispielhafte Querschnittsansicht des Intubationsschlauches nach Figur 1;
- Figur 3: das vergrößerte Detail nach Figur 1,
- Figur 4: eine schematische Darstellung der Komponenten eines Ausführungsbeispiels einer Vorrichtung zur Behandlung von Lungenkrankheiten der vorliegenden Erfindung und
- Figur 5: eine schematische Ansicht eines Trachealkatheters.

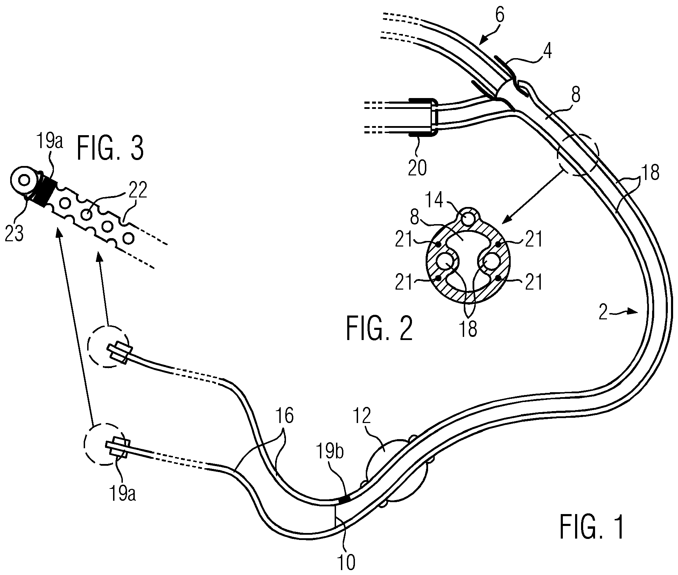

Die Figuren 1 bis 3 verdeutlichen einen Intubationsschlauch 2 mit einem Anschluss 4 zu einem Beatmungsgerät, was vorliegend durch einen Verbindungsschlauch 6 angedeutet ist. Der Intubationsschlauch 2 definiert ein erstes Lumen für die Beatmung, das mit dem Verbindungsschlauch 6 verbunden und mit Bezugszeichen 8 gekennzeichnet ist. Das erste Lumen 8 endet bei Bezugszeichen 10 distal. Unmittelbar benachbart zu diesem distalen Ende 10 befindet sich ein Cuff 12. Diesem Cuff 12 ist ein zweites Lumen 14 zugeordnet, welches parallel zu dem ersten Lumen 8 verlegt ist und proximal mit einem nicht gezeigten Anschluss endet, an den beispielsweise eine Spritze angeschlossen werden kann, um den Cuff 12 durch Einbringen eines Fluids aufzuweiten.

Das distale Ende 10 des ersten Lumens 8 wird von zwei Schlauchstücken 16 überragt, die mit einem dritten Lumen 18 kommunizieren. Wie Figur 2 verdeutlicht, sind zwei solcher dritter Lumen 18 wie auch das zweite Lumen 14 in dem Intubationsschlauch 2 verwirklicht. Mit Bezugszeichen 19 sind jeweils Erregereinrichtungen gekennzeichnet. Bei dem gezeigten Ausführungsbeispiel weist das distale Ende jedes einzelnen Schlauchstücks 16 jeweils eine entsprechende Erregereinrichtung 19a auf. Diese ist vorliegend vergrößert als Manschette um das Lumen des Schlauchstücks 16 dargestellt. In einer tatsächlichen Ausgestaltung der Erfindung ist die Außenumfangsfläche des Schlauchstücks auch auf Höhe der Erregereinrichtung 19 jeweils glatt. Die Erregereinrichtung 19 ist in das Material des Schlauchstücks 16 eingearbeitet. Das gleiche gilt für elektrische Leiter, die in Figur 2 mit Bezugszeichen 21 gekennzeichnet sind, und zu der Erregereinrichtung führen.

Alternativ oder ergänzend kann an dem Ausführungsbeispiel eine Erregereinrichtung 19b vorgesehen sein, die distal in das Material eines Schlauchkörpers eingearbeitet ist, der die Lumen 6, 14, 18 ausbildet und in Figur 2 im Schnitt dargestellt ist.

Bei einer denkbaren Abwandlung wird auf die Schlauchstücke 16 verzichtet. Bei dieser Abwandlung endet der Intubationsschlauch 2 distal mit dem distalen Ende des ersten Lumens 10. Durch die Erregereinrichtung 19b gelöster Schleim kann beispielsweise durch einen herkömmlichen Absaugschlauch entfernt werden, der durch das erste Lumen 8 bis in die Lunge eingeführt wird.

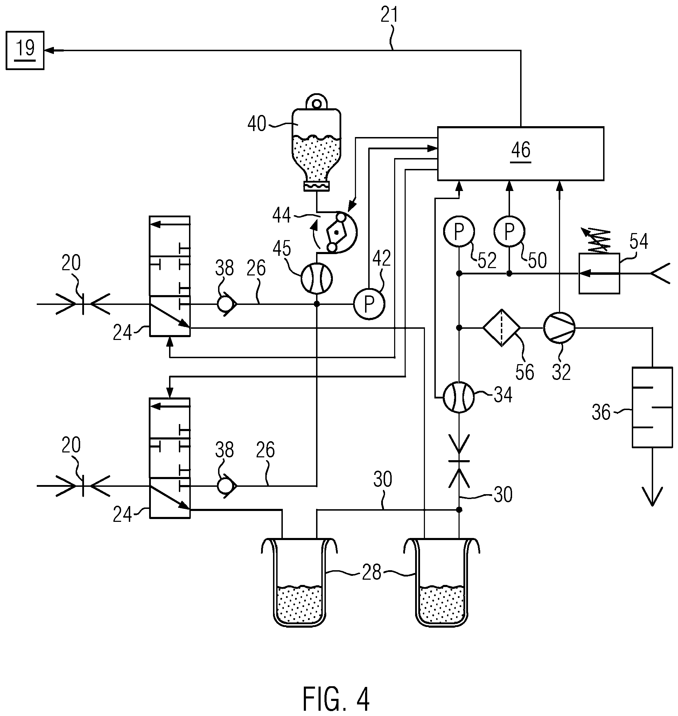

Bei dem gezeigten Ausführungsbeispiel sind an dem proximalen Ende zu dem dritten Lumen 18 Anschlussleitungen 20 vorgesehen, die in der nachstehend unter Bezugnahme auf Figur 4 beschriebenen Weise angeschlossen sind.

Das distale Ende der Schlauchstücke 16 ist in Figur 3 dargestellt. Neben dem freien Ende des Schlauchstücks 16, welches eine Öffnung ausbildet, sind auf dem Umfang verteilt und mit vorbestimmter axialer Länge an dem Schlauchstück 16 eine Vielzahl von Drainageöffnungen 22 ausgebildet. In einem Endbereich jedes Schlauchstücke 16 befindet sich eine Markierung 23 in Form eines in den Schlauchstück 16 eingearbeiteten metallischen Rings zur Detektion der Lage des Schlauchstücks 16 in dem Lungenflügel.

Die Figur 4 zeigt ein Ausführungsbeispiel einer Vorrichtung zur Behandlung von Lungenkrankheiten mit zwei Ventileinrichtungen 24 in Form von 3/3 Wegeventilen, an die jeweils die zuvor erwähnten Anschlussleitungen 20 angeschlossen sind. Auf der gegenüberliegenden Seite sind diese 3/3 Wegeventile mit einer Absaugleitung 30 verbunden, die jeweils zu einem Sammelbehälter 28 führt, die zu einer Saugpume 32 führt, wobei zwischen den Sammelbehältern 28 und der Saugpumpe 32 bei dem gezeigten Ausführungsbeispiel ein Durchflussmesser 34 vorgeschaltet ist, der das Volumen des abgesaugten gasförmigen Fluid misst. Ein Filter 56 ist zwischen den Sammelbehältern 28 und der Saugpumpe 32 angeordnet, der das Volumen entsprechend filtern kann. Die Saugpumpe 32 führt über einen Schalldämpfer 36 und/oder einen antibakteriellen beziehungsweise antiviralen Filter das abgesaugte Gas an die Atmosphäre ab.

Dieses Gas kommt aus der Lunge und wird üblicherweise durch das nicht gezeigte Beatmungsgerät, welches Teil der Vorrichtung ist, über das erste Lumen 8 in die Lunge eingebracht.

Die 3/3 Wegeventile sind ferner an eine Spülleitung 26 angeschlossen, die unter Zwischenschaltung eines Rückschlagventils 38 mit einem Reservoir 40 für Spülflüssigkeit kommuniziert. An einem Abzweig zu den 3/3 Wegeventile ist ein Drucksensor 42 der Spülleitung 26 zugeschaltet, über welchen der Innendruck in der Spülleitung 26 gemessen werden kann. Ein übermäßiger Druckanstieg signalisiert eine Verstopfung der Spülleitung 26 bzw. des dem 3/3 Wegeventil nachgelagerten Schlauchstücks 16. Zwischen dem Drucksensor 42 und in dem Reservoir 40 befindet sich eine Schlauchpumpe 44, die ein Ausführungsbeispiel einer Spülpumpe der vorliegenden Erfindung ist. Denkbar ist ferner, dass die Spülflüssigkeit ohne den Einsatz einer Schlauchpumpe 44 über die Schwerkraft erfolgt. Stromabwärts der Schlauchpumpe 44 ist ein Durchflussmesser 45 angeordnet.

Mit Bezugszeichen 46 ist eine Steuerung gekennzeichnet, die datenmäßig mit der Schlauchpumpe 44, dem Drucksensor 42, dem Durchflussmesser 45 und den beiden 3/3 Wegeventilen verbunden ist und die 3/3 Wegeventile stellt. Die Steuerung 46 ist ferner verbunden mit der Absaugpumpe 32, dem Durchflussmesser 34 und einem mit der Absaugleitung 30 verbundenen Drucksensor 50, der ebenfalls datenmäßig mit der Steuerung 46 verbunden ist. Dem Drucksensor 50 ist ein Manometer 52 zugeordnet, mit dem der Innendruck insbesondere beim manuellen Einstellen des Saugdrucks über den unmittelbar benachbart zu dem Manometer 52 in die Absaugleitung 30 eingebauten Druckregler 54, wobei der Druckregler 54 über die Steuerung 46 reguliert werden kann.

In der Steuerung 46 ist ein Programm hinterlegt, welches die 3/3 Wegeventile jeweils separat ansteuern kann, um diese für den Durchgang eines Fluid zu sperren oder wahlweise mit der Spülleitung 26 bzw. der Absaugleitung 30 zu verbinden. Die Steuerung ist üblicherweise so eingestellt, dass eines der 3/3 Wegeventile den Durchgang von Fluid sperrt, während das andere der 3/3 Wegeventile zunächst Spülflüssigkeit in das Schlauchstücks 16 einleitet und nach einer gewissen vorbestimmten Spüldauer umschaltet, sodass die in die Lunge eingebrachte Flüssigkeit zusammen mit Luft aus der Lunge abgezogen und in dem zugeordneten Sammelbehälter 28 die Flüssigkeit von dem Gas getrennt wird.

Die mit Bezugszeichen 21 in Figur 4 gekennzeichneten elektrischen Leiter gehen von der Steuerung 46 ab. Der Betrieb der Erregereinrichtung 19 erfolgt dementsprechend abhängig von einer in der Steuerung 46 vorgesehenen Logik, die unter anderem auch die Signale der in der Behandlungsvorrichtung vorgesehenen Sensoren verarbeitet und aufgrund einer Analyse dieser Signale die Erregereinrichtung 19 steuert. Es versteht sich, dass zu der Steuerung 46 eine Bedieneinheit vorgesehen ist, mit welcher der Benutzer Betriebsparameter bzw. Spül- und/oder Saugzyklen kombiniert mit dem Betrieb der Erregereinrichtung 19 vorgeben kann.

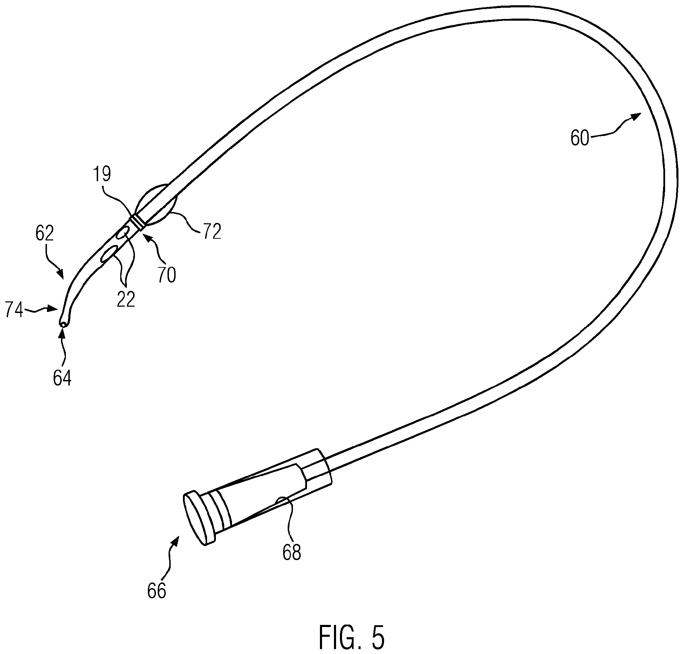

Die Figur 5 zeigt einen Trachealkatheter 60 als Ausführungsbeispiel der vorliegenden Erfindung mit mehreren im Bereich seines distalen Endes 62 vorgesehenen Drainageöffnungen 22. Diese führen zu einem durchgängig in dem Trachealkatheter 60 ausgebildeten Katherterlumen 64, welches von dem distalen Ende 62 bis zu einem proximalen Ende 66 reicht, an welchem der Trachealkatheter 60 ein Anschlusselement 68 beispielsweise für die Anschlussleitung 20 aufweist.

Mit Bezugszeichen 19 ist die Erregereinrichtung gekennzeichnet, die unmittelbar benachbart zu den Drainageöffnungen 22 vorgesehen ist. Diese bildet auch vorliegend eine Markierung 70 aus, die mittels bildgebender Verfahren von außen detektierbar ist, sodass die Lage des distalen Endes 62 in der Lunge detektiert werden kann. Mit Bezugszeichen 72 ist ein Cuff gekennzeichnet, der über ein in dem Trachealkatheter 60 ausgebildetes Lumen aufblasbar ist, um das distale Ende 62 des Trachealkatheters 60 beispielsweise in einem Lungenflügel abzudichten, insbesondere wenn durch den Trachealkatheter 60 nicht nur gesaugt, sondern auch der Lungenflügel gespült werden soll.

Wie Figur 5 verdeutlicht, ist der Trachealkatheter 60 im Bereich seines distalen Endes 62 mit einem gebogenen elastischen Abschnitt 74 versehen, der die Einlassöffnung in das Katherterlumen 64 ausbildet. Dieses freie distale Ende des Trachealkatheters 60 ist gegenüber der üblichen Längserstreckung desselben abgewinkelt. Der gebogene elastische Abschnitt 74 besteht aus einem weichelastischen Material, beispielsweise einem TPE.

Nach einer Variante des in Figur 1 gezeigten Intubationsschlauches 2 kann dieser ohne die Schlauchstücke 16 ausgebildet sein. Die dritten Lumen 18 sind bei dieser Variante hinsichtlich ihres Innendurchmessers an den Außendurchmesser des Trachealkatheters 60 so angepasst, dass die jeweils einer der Trachealkatheter 60 durch eines der dritten Lumen 18 entlang des Intubationsschlauches 2 in die Lunge eingebracht werden kann. Nach dem Einbringen kann beispielsweise zumindest der Cuff 72 das distale Ende des dritten Lumens 18 in der Lunge abdichten. Durch Drehen des Trachealkatheters 60 kann der gebogene elastische Abschnitt 74 in die gewünschte Orientierung gebracht werden, um den Trachealkatheter 60 in einen Lungenflügel einzubringen.

### Bezugszeichenliste

- 2: Intubationsschlauch
- 4: Anschluss
- 6: Verbindungsschlauch
- 8: erstes Lumen
- 10: distales Ende des ersten Lumen
- 12: Cuff
- 14: zweites Lumen
- 16: Schlauchstücke
- 18: drittes Lumen
- 19: Erregereinrichtung
- 20: Anschlussleitung
- 21: elektrischer Leiter
- 22: Drainageöffnung
- 23: Markierung
- 24: Ventileinrichtung
- 26: Spülleitung
- 28: Sammelbehälter
- 30: Absaugleitung
- 32: Saugpumpe
- 34: Durchflussmesser
- 36: Schalldämpfer
- 38: Rückschlagventil
- 40: Reservoir für Spülflüssigkeit
- 42: Drucksensor
- 44: Schlauchpumpe
- 45: Durchflussmesser
- 46: Steuerung
- 50: Drucksensor
- 52: Manometer
- 54: Druckregler
- 56: Filter
- 60: Trachealkatheter
- 62: distales Ende
- 64: Katheterlumen
- 66: proximales Ende
- 68: Anschlusselement
- 70: Markierung
- 72: Cuff
- 74: gebogener elastischer Abschnitt

## Patentansprüche

1. Trachealkatheter (60) mit einem in die Lunge eindringbaren distalen Ende (62) und einem dabei extrakorporal angeordneten proximalen Ende (66), wobei im Bereich des distalen Endes (62) eine das distale Ende (62) umgebende Medium physikalisch anregende Erregereinrichtung (19) vorgesehen ist.

2. Trachealkatheter (60) nach Anspruch 1, **dadurch gekennzeichnet, dass** an dem proximalen Ende ein Schlauchanschluss (68) vorgesehen ist, der mit einem in dem Trachealkatheter (60) ausgebildeten und bis zu dem distalen Ende reichenden Katheterlumen (64) kommuniziert.

3. Trachealkatheter (60) nach Anspruch 1 oder 2 **gekennzeichnet durch** zumindest einen bis zu seinem distalen Ende (62) reichenden Führungsdraht.

4. Trachealkatheter (60) nach einem der vorherigen Ansprüche, **dadurch gekennzeichnet, dass** der Trachealkatheter (60) im Bereich seines distalen Endes (62) einen gebogenen elastischen Abschnitt (74) aufweist.

5. Trachealkatheter (60) nach einem der vorherigen Ansprüche **gekennzeichnet durch** zumindest eine an dem Umfang des Katheters ausgebildete, insbesondere im Bereich des distalen Endes vorgesehene Drainageöffnung (22).

6. Trachealkatheter (60) nach einem der vorherigen Ansprüche **gekennzeichnet durch** eine detektierbare Markierung (60).

7. Trachealkatheter (60) nach einem der vorherigen Ansprüche, **dadurch gekennzeichnet, dass** der Trachealkatheter (60) mit einer Reibkoeffizient-vermindernden Beschichtung, insbesondere einer antihaftenden hydrophilen oder hydrophoben Beschichtung, versehen ist.

8. Intubationsschlauch (2) mit einem ersten Lumen (8) für einen Gasaustausch und einem zweiten Lumen (14) für die Steuerungen eines Cuffs (12), der von einem distalen Ende (10) des ersten Lumens (8) überragt ist, **gekennzeichnet durch** eine dem distalen Ende (10) zugeordnete Erregereinrichtung (19), die ein die Erregereinrichtung (19) umgebendes Medium physikalisch anregt.

9. Intubationsschlauch (2) nach Anspruch 8, **dadurch gekennzeichnet, dass** die Erregereinrichtung (19) an einem Trachealkatheter (60) nach einem der Ansprüche 1 bis 7 angeordnet ist, der beweglich in dem in dem Intubationsschlauch (2), insbesondere einem in dem Intubationsschlauch (2) ausgebildeten dritten Lumen (18) angeordnet ist.

10. Intubationsschlauch (2) nach Anspruch 8 oder 9, **dadurch gekennzeichnet, dass** das erste Lumen (8) mit einer derartigen Länge ausgebildet ist, dass das erste Lumen (8) im Bereich des unteren Endes der Trachea endet.

11. Intubationsschlauch (2) nach einem der Ansprüche 8 bis 10, **dadurch gekennzeichnet, dass** der Trachealkatheter (60) in seiner Verlaufsrichtung axial verschiebbar und vorzugsweise zusätzlich rotierbar ist.

12. Intubationsschlauch (2) nach einem der Ansprüche 8 bis 11, **dadurch gekennzeichnet, dass** der Intubationsschlauch (2) einen Drucksensor aufweist, insbesondere distal im Bereich des Cuffs.

13. Vorrichtung zur Behandlung von Lungenkrankheiten mit einem Beatmungsgerät, das mit einem ersten Lumen (8) eines Intubationsschlauches (2) verbindbar ist, einer Saugpumpe (32), die an zumindest einer Ventileinrichtung (24) angeschlossen ist, die die Saugpumpe (32) mit einer Schnittstelle (20) für einen Saugkanal eines Katethers verbindet, der im Bereich seines distalen Endes mit einer Erregereinrichtung versehen ist, und einer Steuerung (46), die steuerungsmäßig mit der mindestens einen Ventileinrichtung (24), der Saugpumpe (32) und der Erregereinrichtung (19) verbunden ist.

14. Vorrichtung nach Anspruch 13 **gekennzeichnet durch** eine Spülpumpe (44), die steuerungsmäßig mit der mit der Steuerung (46) verbunden ist und an der mindestens einen Ventileinrichtung (24) angeschlossen ist, die die Spülpumpe (44) mit einer Schnittstelle (20) für ein Kathetherlumen (64) eines Trachealkatheters (60) verbindet, und einen in einem Leitungsweg (38) der Spülpumpe (44) angeschlossenen Drucksensor (42), dessen Signal in der Steuerung (46) verarbeitet wird.

15. Vorrichtung nach Anspruch 13 oder 14 **gekennzeichnet durch** einen Intubationsschlauch (2) nach einem der Ansprüche 8 bis 12, dessen erstes Lumen an das Beatmungsgerät und ein an die Ventileinrichtung (24) angeschlossenes drittes Lumen (18) und/oder einen Trachealkatheter (64) aufweist, dessen Katheterlumen (64) an die Ventileinrichtung (24) angeschlossen ist.

16. Verfahren zur Behandlung von Lungenkrankheiten mit einem Trachealkatheter (60) nach einem der Ansprüche 1 bis 7, bei dem das distale Ende (62) des Trachealkatheters (60) in die Lunge eingebracht wird, durch Betrieb der Erregereinrichtung (19) Medium in der Lunge anregt und das dadurch gelöste oder zerkleinerte Medium aus der Lunge entfernt wird.
